# EUROPÄISCHE PATENTSCHRIFT

(11) **EP 1 168 975 B1**
(45) Veröffentlichungstag und Bekanntmachung des Hinweises auf die Patenterteilung: **09.06.2004**
(21) Anmeldenummer: 00920644.2
(22) Anmeldetag: 31.03.2000
(51) Int. Cl.: A61B 19/00, A61B 18/14

(54) **CHIRURGISCHES INSTRUMENT**
SURGICAL INSTRUMENT
INSTRUMENT CHIRURGICAL

(30) Priorität: 01.04.1999 DE 19915060
(43) Veröffentlichungstag der Anmeldung: 09.01.2002
(73) Patentinhaber: Erbe Elektromedizin GmbH., D-72072 Tübingen (DE)
(72) Erfinder: FARIN, Günter, D-72070 Tübingen (DE)
(74) Vertreter: Bohnenberger, Johannes, Dr.
(86) Internationale Anmeldenummer: PCT/EP2000/002906
(87) Internationale Veröffentlichungsnummer: WO 2000/059397

(56) Entgegenhaltungen:
- WO-A-96/09587
- WO-A-98/52479
- US-A- 5 408 409
- US-A- 5 754 741
- US-A- 5 882 206

## Beschreibung

Die Erfindung betrifft ein chirurgisches Instrument, das zumindest teilweise über steuerbare Aktoren, also nicht rein manuell betrieben wird. Ein solches Instrument ist z.B. aus US-A- 5 408 409 bekannt.

In der Chirurgie kommen in zunehmendem Maße Instrumente zum Einsatz, welche relativ komplizierte Arbeitsabläufe durchführen können, wobei ein einziges Instrument benutzt wird. Der Vorteil liegt darin, daß während einer Operation eine Vielzahl von Arbeitsschritten ohne Instrumentenwechsel durchgeführt werden kann. Nachteilig ist diese Entwicklung insofern, als eine Vielzahl von Bedienungselementen ein hohes Maß an Konzentration und auch Übung des Operateurs erfordert.

Der Erfindung liegt die Aufgabe zugrunde, ein chirurgisches Instrument aufzuzeigen, das trotz hoher Komplexität eine Vielzahl von Arbeitsschritten in einer, für den Operateur einfachen Weise durchführbar macht.

Diese Aufgabe wird durch ein chirurgisches Instrument nach Anspruch 1 gelöst.

Die Art des chirurgischen Instrumentes spielt hierbei an sich keine Rolle, wobei die vorteilhaften Auswirkungen der Erfindung aber vor allem dann zum Tragen kommen, wenn mehrere Arbeitsschritte nacheinander durchzuführen sind, die jeweils (oder zumindest teilweise) die Betätigung verschiedener Elemente fordern. Es kann sich also bei den Effektoren, die es "zu bedienen" gilt, um rein mechanische Arbeitseinrichtungen wie Zangen, Haken, Messer, Scheren oder dergleichen sowie Spül- und Saugeinrichtungen handeln oder aber um technisch kompliziertere Einrichtungen wie Ultraschall- oder Kryo-Instrumente sowie Elektroden zum Schneiden oder Koagulieren von Gewebe.

In jedem Fall sind Aktoren vorgesehen, welche die Effektoren (also z. B. eine Schere, eine Spüleinrichtung, eine Vielzahl von Elektroden) zu deren funktionsgerechter Betätigung zugeordnet sind und direkt (z. B. HF-Generatoren) oder über pneumatische, hydraulische oder elektrische oder dergleichen wandler ansteuerbar sind. Die Aktoren können hier auch gemischt derart ausgebildet sein, daß man beispielsweise eine Zangeneinrichtung des chirurgischen Instrumentes manuell betätigt, während eine ihr zugeordnete Spüleinrichtung hydraulisch betätigt wird. Weiterhin ist es auch möglich, manuelle Betätigungseinrichtungen festzusetzen bzw. zu sperren oder freizugeben, je nachdem, welcher Arbeitsschritt gerade durchgeführt werden soll.

Es ist eine Steuerung mit Steuereinrichtungen vorgesehen, in welcher die Schritte mindestens eines Arbeitsprogramms gespeichert sind, nach welchem die Aktoren ansteuerbar sind und welche die Effektoren zur Durchführung von Schritten einer vorwählbaren Funktion betätigen. Es wird hierbei also davon ausgegangen, daß eine bestimmte Reihenfolge von Schritten zur Durchführung einer bestimmten Funktion notwendig ist, wobei diese Schritte in einer ganz bestimmten Reihenfolge durchlaufen werden.

Um nun ein solches Arbeitsprogramm abzuarbeiten, ist eine Betätigungswahleinrichtung mit Betätigungselementen vorgesehen, welche bei Betätigung durch einen Operateur die Steuerung zur sukzessiven Abarbeitung der Schritte ansteuert. Der Operateur muß also nicht für jedes Arbeitsprogramm detailgenau die Schritte bestimmen und "anwählen" , er gibt lediglich das Signal dafür, daß nun der nächste Schritt abgearbeitet wird. Eine solche Betätigungswahleinrichtung kann ein Handschalter, ein Fußschalter oder auch eine akustische Betätigungseinrichtung sein, welche einen vom Operateur ausgeprochenen Befehl erkennt und die Steuerung entsprechend ansteuert.

Vorzugsweise umfaßt die Betätigungswahleinrichtung ein Rückschritt-Betätigungselement, das bei Betätigung durch den Operateur die Steuerung veranlaßt, einen zum momentanen oder zuletzt durchgeführten Schritt (nochmals) abzuarbeiten bzw. zu diesem vorhergehenden Schritt zurückzugehen. Dadurch ist es in einfacher Weise möglich, dann, wenn der Operateur erkennt, daß ein bestimmter Schritt nicht oder noch nicht zum Erfolg geführt hat, innerhalb des Programms zurückzuspringen und die betreffende Aktion zu wiederholen.

weiterhin ist vorzugsweise ein Rücksetz-Betätigungselement vorgesehen, mit dessen Hilfe die Steuerung veranlaßt werden kann, zu einem ersten Schritt oder zur Durchführung der vorwählbaren Funktion zurückzukehren. Das laufende Programm wird also abgebrochen und kann nochmals von vorne begonnen werden.

Vorzugsweise sind verschiedene Sonder-Betätigungselemente vorgesehen, die bei Betätigung durch den Operateur die Steuerung veranlassen, einen vorbestimmbaren Arbeitsschritt durchzuführen. Ein solcher vorbestimmbarer Arbeitsschritt kann beispielsweise ein Zyklus aus Spülen und Absaugen umfassen, der während einer Vielzahl verschiedenartiger Arbeitsabläufe immer wieder auftritt.

Bei einer Ausführungsform der Erfindung umfaßt die Betätigungswahleinrichtung ein Lern-Betätigungselement, das bei Betätigung durch den Operateur die Steuerung veranlaßt, im wesentlichen alle Schritte eines Arbeitsprogrammes nacheinander abzuarbeiten. Vorzugsweise sind zur Verbesserung des Lerneffektes optische und/oder akustische Monitoreinrichtungen vorgesehen, welche die Arbeitsschritte demonstrieren bzw. erläutern. Der Operateur kann also eine Abfolge von Arbeitsschritten, die er während einer Operation durchführen will, zunächst einige Male durchspielen, um dann im Falle der tatsächlichen Operation nicht mehr lange überlegen zu müssen. Eine solche Art der Lernmöglichkeit ist einer reinen Darstellung in einer Videoaufzeichnung weit überlegen, da der Operateur beispielsweise auch miteingreifen kann bzw. miteingreifen muß, wenn das Arbeitsprogramm so gestaltet ist, daß bestimmte Schritte nur durch aktive Manipulation des Operateurs durchgeführt werden. Ein typischer Schritt dieser Art wäre beispielsweise das Betätigen einer Einrichtung, welche dem Gewebe Energie zuführt, um es zu koagulieren, also beispielsweise eine Laservorrichtung oder eine HF-Koagulationsvorrichtung. Es ist also bei dieser bevorzugten Ausführungsform der Erfindung vorgesehen, das Lernprogramm interaktiv auszugestalten und dem Operateur vor allem zu zeigen, welche Schritte nacheinander geschehen werden.

Diese Lernfunktion ist besonders vorteilhaft dann, wenn die Speichereinrichtungen zur Eingabe und Abfrage vom Benutzer vorgegebener Arbeitsprogramme ausgestattet ist. Der Operateur kann in diesem Falle besonders einfach ein nach seinem Dafürhalten optimales Arbeitsprogramm aufstellen, wobei er sich sowohl vorab eingespeicherter Arbeitsanweisungen bedienen und diese gruppenweise zusammenfügen oder aber selbst besondere Arbeitsschritte einspeichern kann, die seinem "Arbeitsstil" entsprechen. Die vorgegebenen Programmabläufe können dann vom Operateur beliebig oft kontrolliert und gegebenenfalls korrigiert werden. Vorteilhafterweise ist der Speicher (bzw. ein Teil hiervon) auswechselbar ausgebildet, so daß mehrere Personen mit ein und demselben Gerät arbeiten und "ihre" Programme bei sich aufbewahren können, was eine erheblich verbesserte Dokumentation ermöglicht und gleichzeitig sicherstellt, daß das chirurgische Instrument bzw. der zu ihm gehörige PC nicht überfrachtet wird.

Nachfolgend wird eine Ausführungsform der Erfindung anhand eines Beispiels beschrieben, welches ein besonderes mechanisch-elektrochirurgisches Instrument umfaßt. Es sei aber an dieser Stelle nochmals betont, daß die Erfindung nicht auf ein spezielles chirurgisches Instrument beschränkt ist, sondern vielmehr eine Vielzahl von insbesondere multifunktionalen chirurgischen Instrumenten betrifft bzw. auf diese angewendet werden soll.

In den beiliegenden Abbildungen zeigt
- Fig. 1: eine perspektivische Darstellung eines Endstückes eines Präparationsinstrumentes,
- Fig. 2: eine schematisierte Darstellung des Instrumentes nach Fig. 1 in einer ersten geöffneten Position,
- Fig. 3: das Instrument nach Fig. 2, jedoch in einer zweiten geöffneten Position und
- Fig. 4: ein schematisiertes Blockschaltbild zur Erläuterung der Erfindung.

In der nachfolgenden Beschreibung werden für gleiche und gleich wirkende Teile dieselben Bezugsziffern verwendet.

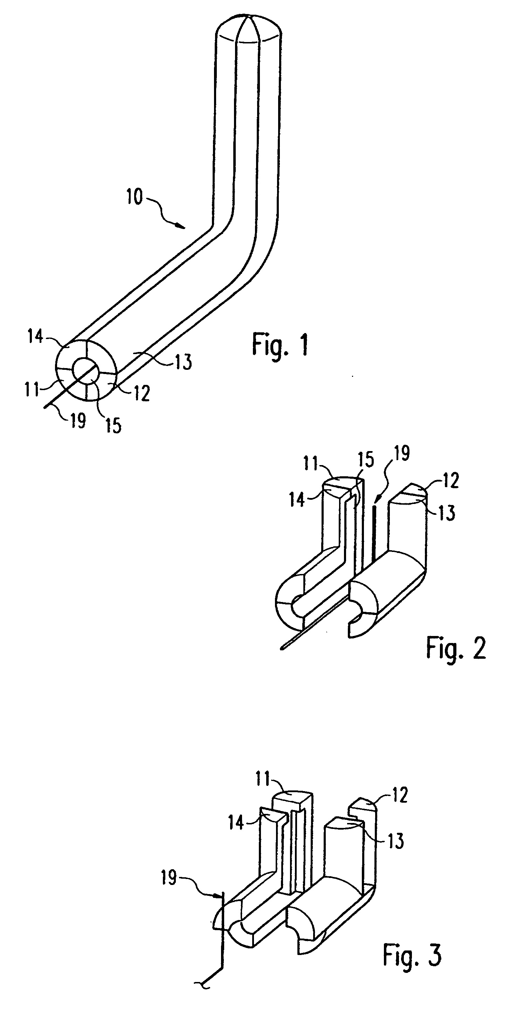

In Fig. 1 ist in perspektivischer Ansicht ein Endabschnitt eines multifunktionalen Instrumentes gezeigt, das in diesem Fall einen Präparierhaken 10 zeigt, der aus vier Teilhaken 11, 12, 13 und 14 besteht. Die vier Teilhaken 11-14 sind so geformt, daß sie aneinander gelegt werden können und dann eine im wesentlichen glatte Außenfläche bilden.

Die Teilhaken 11 bis 14 bilden weiterhin in ihrem zusammengelegten Zustand (wie in Fig. 1 gezeigt) eine innere Ausnehmung 15, in welcher eine Schneidelektrode 19 sitzt. Die Teilhaken 11-14 sind nun derart mit hier nicht gezeigten Aktoren verbunden, daß sie in den in den Figuren 2 und 3 gezeigten Weisen bewegbar sind. Insbesondere können die Teilhaken 11-14 derart auseinander gefahren werden, daß sie zwischeneinander Spalte bilden, deren Ebenen senkrecht zueinander stehen. Die Schneidelektrode 19 wiederum ist in Relation zu den Teilhaken 11-14 verschiebbar angeordnet.

Das Instrument kann nun (beispielsweise) so wie in Fig. 1 gezeigt benutzt werden, dient also als (einfacher) mechanischer Präparierhaken. Wenn dann beispielsweise ein Blutgefäß freigelegt wurde, das nun durchtrennt werden soll, so werden die Teilhaken 11 so auseinandergefahren, wie dies in Fig. 3 gezeigt ist. Die Schneidelektrode 19, die bis zu diesem Zeitpunkt innerhalb der Teilhaken 11-14 bzw. in der zwischen diesen liegenden Ausnehmung 15 geschützt war, wird nun zurückgezogen (siehe Fig. 3). Nun kann man das frei präparierte Blutgefäß so zwischen die Teilhaken 11-14 einführen, daß es (in Fig. 3 von links nach rechts) durch diese hindurchläuft, um sodann die Teilhaken 11 und 14 bzw. 12 und 13 aufeinander zuzubewegen, so daß sie das Blutgefäß zwischeneinander einklemmen. In diesem Zustand wird nun ein Koagulationsstrom derart den aus Metall ausgebildeten Teilhaken 11 bis 14 zugeführt, daß einerseits ein Koagulationsstrom zwischen dem Teilhaken 11 und dem Teilhaken 14 und andererseits ein Koagulationsstrom zwischen dem Teilhaken 12 und dem Teilhaken 13 fließt und dabei das zwischen diesen Teilhaken liegende Gewebe koaguliert. Damit ist sichergestellt, daß nun bei einem Durchtrennen des Gefäßes zwischen den Koagulationsstellen keine Blutung auftritt. Nun wird die Schneidelektrode 19 in Richtung auf das Gewebe bewegt. Es wird weiterhin die Schneidelektrode 19 mit einem Pol eines Hochfrequenzgenerators verbunden, dessen anderer Pol mit den Teilhaken 11-14 (oder zumindest mit einem dieser Teilhaken) verbunden ist. Sobald die Schneidelektrode 19 auf den zwischen den Teilhaken 11, 14, bzw. 12, 13 eingeklemmten Gewebeabschnitt auftrifft, fließt ein Schneidstrom, der das Gewebe bzw. das Blutgefäß in diesem Bereich durchtrennt. Nun können die Teilhaken 11-14 wieder auseinandergefahren werden, so daß sie die voneinander getrennten Abschnitte des Blutgefäßes freigeben. Der Haken wird nun bewegt, so daß die freien Enden aus den Spalten zwischen den Teilhaken 11-14 herausrutschen, woraufhin die Elektrode 19 wieder in ihre Parkposition (siehe Fig. 2) gefahren und die Teilhaken 11-14 wieder zusammengelegt werden, um den in Fig. 1 gezeigten Präparierhaken 10 zu bilden.

Der oben beschriebene Vorgang erfordert ersichtlich eine Reihe von verschiedenen, nacheinander ablaufenden Verfahrensschritten.

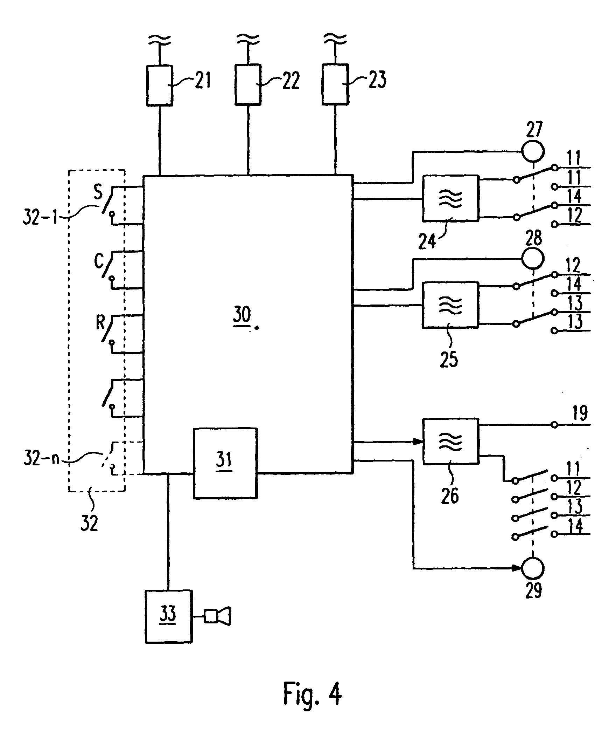

Um diese verfahrensschritte nun in vereinfachter Weise durchführen zu können, dient die in Fig. 4 schematisiert gezeigte Anordnung. Diese umfaßt Aktoren vielfältiger Art. Zunächst sind hier drei Antriebe 21, 22 und 23 zu nennen, von welchen der erste Antrieb 21 die beiden Teilhaken 13 und 14 gegenüber den beiden Teilhaken 11 und 12 verschiebt, während der zweite Antrieb 22 die Teilhaken 11 und 14 gegenüber den Teilhaken 12 und 13 auseinanderklappt (wie dies in Fig. 2 gezeigt ist). Der dritte Antrieb 23 dient zur Bewegung der Schneidelektrode 19 aus der in Fig. 2 gezeigten Stellung in die in Fig. 3 gezeigte Stellung und wieder zurück. Die Antriebe 21-23 werden von einer Steuerung 30 angesteuert.

An der Steuerung 30 liegen weiterhin drei HF-Generatoren 24, 25 und 26, von denen der erste Generator 24 über einen Schalter 27, der ebenfalls von der Steuerung 30 angesteuert wird, wahlweise mit den Teilhaken 11 und 14 (bzw. mit dort angebrachten Elektroden) oder mit den Teilhaken 11 und 12 verbunden werden kann. Der zweite Generator 25 kann ebenfalls über einen gesteuerten Schalter 28 wahlweise mit den Teilhaken 12 und 13 oder 14 und 13 verbunden werden. Auf diese Weise ist es möglich, einen Koagulationsstrom zwischen den Teilhaken 11 und 14 oder den Teilhaken 11 und 12 bzw. zwischen den Teilhaken 12 und 13 oder 14 und 13 fließen zu lassen, um dazwischen eingeklemmtes Gewebe zu koagulieren.

Der dritte HF-Generator 26 ist einerseits mit der Elektrode 19 und andererseits über einen Schalter 29 gesteuert mit allen Teilhaken 11, 12, 13 und 14 verbindbar. Um den oben genannten Schneidvorgang durchzuführen, wird der Schalter 29 geschlossen, so daß ein Schneidstrom zwischen der Schneidelektrode 19 und den Teilhaken 11-14 in das Gewebe eingeleitet wird.

Die Stellung der Schalter kann auch selbsttätig in Abhängigkeit von der Position der Teilhaken 11-14 eingestellt werden.

Zur Betätigung der Steuerung 30 ist eine Betätigungswahleinrichtung 32 vorgesehen, welche eine Vielzahl von Schaltern 32-1 bis 32-n umfaßt. Von diesen Schaltern sind in Fig. 4 drei Schalter mit S, C und R bezeichnet, deren Funktion in der nachfolgenden Beschreibung erläutert wird.

weiterhin ist in der Steuerung 30 ein Speicher 31 vorgesehen, der (zumindest teilweise) vorzugsweise abgenommen und gegen einen anderen (im wesentlichen gleich aufgebauten) Speicher 31 ausgetauscht werden kann.

Die Steuerung 30 weist schließlich auch eine Monitoreinrichtung 33 auf, die optische und akustische Informationen wiedergeben kann.

Nachfolgend wird erläutert, wie die in Fig. 4 gezeigte Anordnung dazu verwendet werden kann, die oben beschriebenen Arbeitsschritte zum Durchtrennen von Gewebe unter gleichzeitiger Durchführung einer Hämostase zu bewerkstelligen.

Zunächst wird ein im Speicher 31 abgespeichertes Programm "Trennen" aufgerufen. Hierbei befindet sich das Instrument noch in der in Fig. 1 gezeigten Stellung, in welcher die Teilhaken 11-14 zur Bildung des Hakens 10 zusammengelegt sind.

In einem ersten Schritt wird die Set-Taste "S" betätigt. Daraufhin steuert die Steuerung 30 den Antrieb 21 so an, daß ein Spalt zwischen den Teilhaken 11 und 14 bzw. 12 und 13 entsteht, in welchen Gewebe eingeladen wird.

Bei einer neuerlichen Betätigung des Set-Schalters "S" werden die Teilhaken 11 und 14 gegenüber den Teilhaken 12 und 13 durch den Antrieb 22 auseinandergefahren und durch Ansteuerung des Antriebs 23 die Schneidelektrode 19 zurückgezogen (siehe Fig. 3).

In einem dritten Arbeitsschritt wird bei erneuter Betätigung des Set-Schalters "S" der Antrieb 21 wieder betätigt, so daß die Teilhaken 11 und 14 bzw. 12 und 13 wieder aufeinander zu bewegt werden, um das zwischen ihnen liegende Gewebe (vorzugsweise mit definierter Kraft) einzuklemmen.

Nun kann in einem weiteren Arbeitsschritt und zwar entweder durch erneute Betätigung der Set-Taste "S" oder aber durch Betätigung eines getrennten Schalters ein Koagulationsstrom in die beiden erfaßten Gewebeabschnitte eingeleitet werden.

Bei einer erneuten Betätigung des Set-Schalters "S" wird nun die Schneidelektrode 19 mittels des Antriebs 23 zum Gewebe hin und unter Aktivierung der Schneidspannung durch das Gewebe hindurchgeführt. Auch hier kann wieder die Zuführung der Schneidspannung über eine gesonderte Taste oder einen Fußschalter oder dergleichen erfolgen.

In einem weiteren Schritt werden nun die Schneidelektrode 19 zurückgezogen und die Teilhaken 11 und 14 bzw. 12 und 13 zur Öffnung des Spaltes bzw. zum Freigeben des eingeklemmten Gewebes auseinandergefahren. Sobald das Gewebe zwischen den Teilhaken 11-14 entfernt wurde, wird nun durch eine erneute Betätigung des Set-Schalters "S" die Schneidelektrode 19 wieder zurückgefahren und die Teilhaken 11-14 werden wieder zusammengelegt, so daß die in Fig. 1 gezeigte Stellung erreicht ist.

Aus obigem geht hervor, daß eine Vielzahl von Teilschritten, die doch einigen Manipulieraufwand bedeuten, durch einfaches wiederholtes Betätigen eines einzigen Schalters nacheinander abgearbeitet werden können.

Der Cancel-Schalter "C" veranlaßt die Steuerung 30 jeweils einen Schritt in der vorgegebenen Reihenfolge zurückzugehen, wenn aus irgendwelchen Gründen das Arbeitsprogramm abgeändert werden muß. Der Reset-Schalter "R" führt bei Betätigung dazu, daß das Instrument in seiner Ausgangsstellung (Fig. 1) zurückgesetzt wird.

Es ist ersichtlich weiterhin möglich, zusätzliche Schalter und Betätigungseinrichtungen vorzusehen, um zusätzliche Funktionen durchzuführen und zwar insbesondere solche, deren Notwendigkeit nicht unbedingt oder direkt von einem bestimmten Arbeitsschritt abhängt. Eine solche Sonderfunktion kann beispielsweise das Zuführen von Spülflüssigkeit (gegebenenfalls unter nachfolgendem Absaugen) oder das Absaugen von Körperflüssigkeit sein.

Im Speicher 31 können nun ein oder mehrere Programme zu bestimmten Funktionsabläufen gespeichert sein, die wahlweise abrufbar sind. Um die gespeicherten Programme möglichst leicht kennenlernen zu können oder auch um vom Operateur eingefügte Änderungen (zusätzliche Verfahrensschritte oder Fortlassen von Verfahrensschritten) besser nachvollziehbar zu machen, kann die Steuerung 30 veranlaßt werden, die Programmschritte automatisch abzuarbeiten und dabei gleichzeitig mit den Programmschritten korrelierte Informationen über die Monitoreinrichtungen 33 wiederzugeben.

### Bezugszeichenliste

- 10: Haken
- 11-14: Teilhaken
- 15: Ausnehmung
- 19: Schneidelektrode
- 21-23: Antrieb
- 24-26: HF-Generator
- 27-29: Schalter
- 30: Steuerung
- 31: Speicher
- 32: Betätigungswahleinrichtung
- 33: Monitoreinrichtung

## Patentansprüche

1. Chirurgisches Instrument mit einer Betätigungseinrichtung, umfassend mehrere Arbeitseinrichtungen wie Zangen, Haken, Ultraschall- oder Kryo-Instrumente, Spül- oder Saugeinrichtungen, Elektroden zum Schneiden oder Koagulieren oder dergleichen Effektoren (10-14; 19), Aktoren (21-29), welche den Effektoren (10-14; 19) zu deren funktionsgerechter Betätigung zugeordnet sind und direkt oder über pneumatische, hydraulische oder elektrische oder dergleichen Wandler ansteuerbar sind,
eine Steuerung (30) mit Speichereinrichtungen (31) zum Speichern von Schritten mindestens eines Arbeitsprogramms, nach welchem verschiedene Aktoren (21-29) nacheinander ansteuerbar sind und welche die Effektoren (10-14; 19) zur Durchführung von Schritten einer vorwählbaren Funktion betätigen, und
eine Betätigungswahleinrichtung (32) mit Betätigungselementen (32-1; 32-n), welche bei Betätigung durch einen Operateur die Steuerung (30) zur sukzessiven Abarbeitung der Schritte ansteuert.

2. Chirurgisches Instrument nach Anspruch 1,
**dadurch gekennzeichnet,**
**daß** die Betätigungswahleinrichtung (32) ein Rückschritt-Betätigungselement (C) umfaßt, das bei Betätigung durch den Operateur die Steuerung (30) veranlaßt, einen zum momentanen oder zuletzt durchgeführten Schritt vorhergehenden Schritt abzuarbeiten.

3. Chirurgisches Instrument nach Anspruch 1,
**dadurch gekennzeichnet,**
**daß** die Betätigungswahleinrichtung (32) ein Rücksetz-Betätigungselement (R) umfaßt, das bei Betätigung durch den Operateur die Steuerung (30) veranlaßt, zu einem ersten Schritt vor oder zur Durchführung der vorwählbaren Funktion zurückzukehren.

4. Chirurgisches Instrument nach Anspruch 1,
**dadurch gekennzeichnet,**
**daß** die Betätigungswahleinrichtung (32) mindestens ein Sonder-Betätigungselement umfaßt, das bei Betätigung durch den Operateur die Steuerung veranlaßt, einen vorbestimmbaren Arbeitsschritt oder eine Gruppe von Arbeitsschritten durchzuführen.

5. Chirurgisches Instrument nach Anspruch 1,
**dadurch gekennzeichnet,**
**daß** die Betätigungswahleinrichtung (32) ein Lern-Betätigungselement umfaßt, das bei Betätigung durch den Operateur die Steuerung (30) veranlaßt, im wesentlichen alle Schritte eines Arbeitsprogrammes nacheinander abzuarbeiten.

6. Chirurgisches Instrument nach Anspruch 5,
**gekennzeichnet durch**
eine optische und/oder akustische Monitoreinrichtung (33), welche die Arbeitsschritte demonstriert und/oder erläutert.

7. Chirurgisches Instrument nach einem der vorhergehenden Ansprüche,
**dadurch gekennzeichnet,**
**daß** die Speichereinrichtung (31) auswechselbar ausgestaltet und zur Vorgabe bestimmter Arbeitsprogramme vorgefertigt ist.

8. Chirurgisches Instrument nach einem der vorhergehenden Ansprüche,
**dadurch gekennzeichnet,**
**daß** die Speichereinrichtung (31) zur Eingabe und Abfrage vom Benutzer vorgegebene Arbeitsprogramme ausgestaltet ist.

## Claims

1. A surgical instrument with an actuating device, comprising a plurality of operating devices, such as forceps hooks, ultrasonic or cryogenic instruments, flushing or suction devices, electrodes for the cutting or coagulation, or like actuators (10-14;19), control elements (21-29) which are associated with the actuators (10-14;19) for the operationally suitable actuation thereof and which can be activated directly or via pneumatic, hydraulic or electric or like transducers,
a control system (30) with a memory means (31) for storing steps of at least one operating program, according to which different control elements (21-29) can be successively controlled and actuate the actuators (10-14;19) to perform steps of a preselectable function, and
an actuating selection device (32) with actuating elements (32-1;32-n) which upon actuation by a surgeon triggers the control system (30) for successive execution of the steps.

2. A surgical instrument according to Claim 1, **characterised in that** the actuating selection device (32) comprises a regressive actuating element (C) which upon actuating by the surgeon induces the control system (30) to execute a step preceding the present step or the step last performed.

3. A surgical instrument according to Claim 1, **characterised in that** the actuating selection device (32) comprises a resetting actuating element (R) which upon actuation by the surgeon induces the control system (30) to return to a first step or to carry out the preselectable function.

4. A surgical instrument according to Claim 1, **characterised in that** the actuating selection device (32) comprises a special actuating element which upon actuation by the surgeon induces the control system to carry out a predeterminable operating step or a group of operating steps.

5. A surgical instrument according to Claim 1, **characterised in that** the actuating selection device (32) comprises a learning actuating element which upon actuation by the operator induces the control system (30) to execute substantially all the steps of an operating program.

6. A surgical instrument according to Claim 5, **characterised by** a optical and/or acoustic monitoring device (33) which demonstrates and/or explains the operating steps.

7. A surgical instrument according to any one of the preceding Claims, **characterised in that** the memory means (31) is designed to be interchangeable and is prefabricated to preselect certain operating programs.

8. A surgical instrument according to any one of the preceding Claims, **characterised in that** the memory means (31) is designed for the input and interrogation of preselected operating programs by the user.

## Revendications

1. Instrument chirurgical avec un système d'actionnement, comprenant
- plusieurs équipements opératoires tels que pinces, crochets, instruments à ultrasons ou cryogéniques, dispositifs de lavage ou d'aspiration, électrodes pour la coupe ou la coagulation ou effecteurs analogues (10 - 14 ; 19), des acteurs (21 - 29) qui sont associés aux effecteurs (10 - 14 ; 19) pour leur fonctionnement conforme et peuvent être attaqués directement par l'intermédiaire de convertisseurs pneumatiques, hydrauliques ou électriques, ou autres,
- une commande (30) avec des dispositifs de mémorisation (31) pour mémoriser des phases d'au moins un programme opératoire, selon lequel différents acteurs (21 - 29) peuvent être attaqués les uns après les autres et actionnent les effecteurs (10 - 14 ; 19) pour la réalisation de phases d'une fonction pré-sélectionnable, et
- un système d'actionnement sélectif (32) avec des éléments d'actionnement (32-1 ; 32-n) qui attaque, lors de son actionnement par un chirurgien, la commande (30) pour l'exécution successive des phases.

2. Instrument chirurgical suivant la revendication 1, **caractérisé en ce que** le système d'actionnement sélectif (32) comprend un élément d'actionnement de retour (C), qui amène lors de son actionnement par le chirurgien la commande (30) à exécuter une phase réalisée momentanément ou dernièrement ou une phase précédente.

3. Instrument chirurgical suivant la revendication 1, **caractérisé en ce que** le système d'actionnement sélectif (32) comprend un élément d'actionnement de remise à zéro (R), qui amène lors de son actionnement par le chirurgien la commande (30) à revenir à une première phase ou à la réalisation de la fonction pré-sélectionnable.

4. Instrument chirurgical suivant la revendication 1, **caractérisé en ce que** le système d'actionnement sélectif (32) comprend au moins un élément d'actionnement spécial, qui amène lors de son actionnement par le chirurgien la commande à réaliser une phase opératoire prédéfinissable ou un groupe de phases opératoires.

5. Instrument chirurgical suivant la revendication 1, **caractérisé en ce que** le système d'actionnement sélectif (32) comprend un élément d'actionnement de formation, qui amène lors de son actionnement par le chirurgien la commande (30) à exécuter essentiellement toutes les phases d'un programme opératoire les unes après les autres.

6. Instrument chirurgical suivant la revendication 5, **caractérisé par** un système moniteur (33) optique et/ou acoustique, qui fait la démonstration des phases de travail et/ou les explique.

7. Instrument chirurgical suivant l'une des revendications précédentes, **caractérisé en ce que** le dispositif de mémorisation (31) est configuré de façon interchangeable et est préfabriqué pour la consigne de programmes opératoires définis.

8. Instrument chirurgical suivant l'une des revendications précédentes, **caractérisé en ce que** le dispositif de mémorisation (31) est configuré pour l'introduction et l'interrogation de programmes opératoires prédéfinis par l'utilisateur.
